# EUROPEAN PATENT SPECIFICATION

(11) **EP 2 686 046 B1**
(45) Date of publication and mention of the grant of the patent: **01.05.2019**
(21) Application number: 11861021.1
(22) Date of filing: 17.03.2011
(51) Int. Cl.: A61M 5/315

(54) **MEDICAL INJECTOR WITH ROTATABLE RATCHETING PLUNGER**
MEDIZINISCHER INJEKTOR MIT ROTIERBAREM SPERRKLINKENKOLBEN
INJECTEUR MÉDICAL COMPORTANT UN PISTON PLONGEUR D'ENCLIQUETAGE ROTATIF

(43) Date of publication of application: 22.01.2014
(60) Divisional application: 16158107.9
(73) Proprietor: Becton, Dickinson and Company, Franklin Lakes, NJ 07417-1880 (US)
(72) Inventor: CRONENBERG, Richard, Mahwah, NJ 07430 (US); WU, Haiming, North Attleboro, MA 02760 (US)
(74) Representative: dompatent von Kreisler Selting Werner - Partnerschaft von Patent- und Rechtsanwälten mbB
(86) International application number: PCT/US2011/000492
(87) International publication number: WO 2012/125132

(56) References cited:
- EP-A2- 0 272 035
- WO-A1-94/06494
- WO-A1-94/13339
- WO-A1-2011/039215
- WO-A2-03/020347
- WO-A2-2010/033770
- WO-A2-2010/033770
- US-A- 5 224 936
- US-A- 5 224 936
- US-A1- 2002 007 142
- US-A1- 2007 233 004
- US-A1- 2008 071 227
- US-A1- 2011 034 870

## Description

### FIELD OF THE INVENTION

This invention relates to displaceable medical injector plungers and, more particularly, to ratcheting, rotatable medical injector plungers.

### BACKGROUND OF THE INVENTION

Medical injectors are well known in the art, including syringes and pen injectors. Medical injectors typically include a plunger for advancing one or more stoppers in delivering a medicament during an injection. Although it is known in the prior art to provide syringe plungers with teeth or other features to prevent retraction and re-use after an initial injection, syringe plungers are typically actuated through direct application of linear force. Dose size is a direct function of plunger displacement. It may be difficult to control linear displacement of the plunger, thus resulting in difficulty over control of dose size.

As for pen injectors, a lead screw or rotating plunger is provided which is mechanically coupled to a dose-setting knob or other actuator through a series of mechanical connections. The typical pen injector mechanism is fairly complex and consists of multiple cooperating parts. For costs reasons and simplicity of use, a minimum number of working parts is desired.

The features described within the preamble of claim 1 are disclosed in WO 94/13339, WO94/06494A1 and WO2010/033770A2.

### SUMMARY OF EMBODIMENTS OF THE INVENTION

Accordingly, it is an aspect of the present invention to provide a plunger for a medical injector which may be controllably advanced with a minimum number of cooperating parts.

The invention is defined by features of claim 1 defining a medical injector and a method of the reconstituting a lyophilized medicament according to claim 6.

The foregoing and/or other aspects of the present invention are achieved by providing a medical injector including a body having a distal end and a proximal end, and a displaceable plunger disposed in the body. The plunger includes a plurality of spaced-apart ratchet teeth disposed along the length thereof. At least one indexer is provided and is formed to engage the plunger, wherein the indexer is configured to allow the plunger to displace distally toward a distal end of the body but not proximally toward a proximal end of the body. The medical injector also includes an actuator having an engagement portion formed to engage one or more of the ratchet teeth. The actuator is displaceable to a ready state, the engagement portion being displaced proximally relative to the plunger with the actuator being displaced to the ready state. The indexer prevents proximal movement of the plunger thereby allowing the engagement portion to bypass one or more of the ratchet teeth with the actuator being displaced to the ready state. In addition, the actuator is displaceable from the ready state to cause actuation of the medical injector. The displacement from the ready state causes distal displacement of the engagement portion with the engagement portion engaging one or more of the ratchet teeth and causing distal displacement of the plunger with the engagement portion. The plunger is slidably displaceable relative to the body, and rotatable relative to the body to selectively engage and disengage ratchet teeth engaged with the indexer and the actuator.

The foregoing and/or other aspects of the present invention are also achieved by providing a medical injector including a body having a distal end and a proximal end, and a plunger displaceably disposed in the body. The plunger includes a plurality of spaced-apart ratchet teeth disposed along the length thereof. The plunger selectively displaces a stopper to dispense a medicament from said medical injector. The medical injector also includes an indexer disposed within the body to engage the plunger to permit distal displacement of the plunger and substantially prevent proximal displacement of the plunger, and an actuator having an engagement portion to engage the plunger to permit proximal displacement of the actuator relative to the plunger and substantially prevent distal displacement of the actuator relative to the plunger. Upon proximal displacement of the actuator relative to the body to a ready state, one or more of the ratchet teeth bypass the engagement portion to proximally displace the actuator relative to the plunger. Upon distal displacement of the actuator relative to the body from the ready state, the actuator engages one or more of the ratchet teeth to distally displace the plunger relative to the indexer to distally displace the stopper to dispense medicament from the medical injector. The plunger is slidably displaceable relative to the body, and rotatable relative to the body to selectively engage and disengage ratchet teeth engaged with the indexer and the actuator.

The foregoing and/or other aspects of the present invention are also achieved by providing a medical injector including a body having a distal end and a proximal end, and a plunger displaceably disposed in the body. The plunger includes a plurality of spaced-apart ratchet teeth disposed along the length thereof. The plunger selectively displaces a stopper to dispense a medicament from said medical injector. The medical injector also includes an indexer disposed within the body to engage the plunger to permit distal displacement of the plunger and substantially prevent proximal displacement of the plunger. The medical injector additionally includes actuating means for actuating the medical device, the actuating means having engagement means for engaging the plunger to permit proximal displacement of the actuating means relative to the plunger and substantially prevent distal displacement of the actuating means relative to the plunger. Upon proximal displacement of the actuating means relative to the body to a ready state, one or more of the ratchet teeth bypass the engagement means to proximally displace the actuating means relative to the plunger. Upon distal displacement of the actuating means relative to the body from the ready state, the actuating means engages one or more of the ratchet teeth to distally displace the plunger relative to the indexer to distally displace the stopper to dispense medicament from the medical injector. The plunger is rotatable relative to the body to selectively engage and disengage ratchet teeth engaged with the indexer and the actuator means.

The foregoing and/or other aspects of the present invention are also achieved by providing a method of reconstituting a lyophilized medicament using a medical injector having a body with a plunger disposed therein. The method includes the operations of connecting a medicament container containing the lyophilized medicament to an end of the body so that a double ended needle communicates between the medicament container and a diluent containing cartridge disposed within the body, rotating the plunger to disengage ratchet teeth on the plunger from the body, displacing the plunger to eject diluent into the medicament container, displacing the plunger to draw reconstituted medicament into the cartridge, and rotating the plunger to engage the ratchet teeth of the plunger with the body.

Additional and/or other aspects and advantages of the present invention will be set forth in part in the description that follows and, in part, will be apparent from the description, or may be learned by practice of the invention.

### BRIEF DESCRIPTION OF THE DRAWINGS

The above and/or other aspects and advantages of embodiments of the invention will become apparent and more readily appreciated from the following detailed description, taken in conjunction with the accompanying drawings, in which
Figure 1 is a perspective view of a medical injector in accordance with an embodiment of present the invention;
Figure 2 is partial perspective view of a plunger useable with the injector of Figure 1;
Figure 3 is a partial cross-sectional view taken along line 3-3 of Figure 2;
Figures 4-6 are perspective partial cross-sectional views illustrating operation of the medical injector of Figure 1;
Figures 7 and 8 are perspective views respectively illustrating two rockers useable with the injector of Figure 1;
Figures 9-11 are perspective partial cross-sectional views illustrating operation of a medical injector using the rocker of Figure 7;
Figures 12 and 13 are perspective partial cross-sectional views illustrating the use of a multi-link rocker
Figure 14 shows a dose counter useable with an embodiment of the present invention;
Figure 15 is a partial perspective view of a pivotable actuator useable with the plunger of Figure 2;
Figures 16 and 17 are perspective partial cross-sectional views illustrating use of the pivotable actuator of Figure 15,
Figure 18 is a cross-sectional view of a medical injector;
Figure 19 is a perspective cross-sectional view of the medical injector of Figure 18 taken along line 19-19 of Figure 18;
Figure 20 is a perspective view of a medical injector according to another embodiment of the present invention;
Figure 21 is a perspective view of a plunger useable with the medical injector of Figure 20;
Figure 22 is a cross-sectional view of the medical injector of Figure 21 taken along line 22-22 of Figure 21;
Figure 23 is a perspective cross-sectional view illustrating operation of the medical injector of Figure 21; and
Figure 24 is a perspective view of another embodiment of a plunger useable with the medical injector of Figure 20.

### DETAILED DESCRIPTION OF EMBODIMENTS OF THE INVENTION

Reference will now be made in detail to embodiments of the present invention, examples of which are illustrated in the accompanying drawings, wherein like reference numerals refer to the like elements throughout. The embodiments described herein exemplify, but do not limit, the present invention by referring to the drawings. As will be understood by one skilled in the art, terms such as up, down, bottom, and top are relative, and are employed to aid illustration, but are not limiting.

With reference to the figures, a medical injector 10 is shown having a ratchetable plunger 12 provided therewith. As will be appreciated by those skilled in the art, the medical injector 10 may be of various forms, including being a syringe or pen injector. In accordance with an embodiment of the present invention, the medical injector 10 is particularly well-suited for administering at least one fixed dose, and is even better suited for administering a series of fixed doses. The medical injector 10 may be configured in any way known to be compatible with the plunger 12 as described herein. The medical injector 10 may include a reservoir 14 for accommodating an injectable medicament, which may be a drug cartridge, or which may be formed directly in the medical injector 10. The reservoir 14 may have one or more stoppers 16 associated therewith as known in the art. The medical injector 10 may also be provided with a needle 18 for injection which may be removably attached or affixed to the medical injector 10 such as in a "staked" arrangement.

The plunger 12 is elongated and generally flat. A plurality of spaced-apart ratchet teeth 20 are disposed along the length of the plunger 12. In a preferred arrangement, the plunger 12 includes a plate-shaped body 22 having opposing first and second faces 24, 26. The ratchet teeth 20 are disposed on the first face 24 and, in a further preferred arrangement, also on the second face 26. Preferably, the ratchet teeth 20 on the first and second faces 24, 26 are axially aligned along the length of the plunger 12.

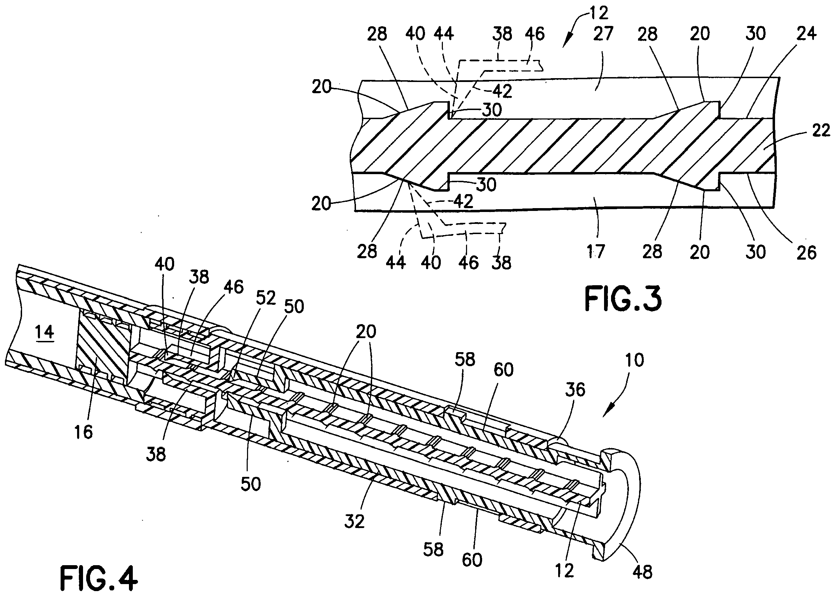

The ratchet teeth 20 are configured to permit unidirectional movement of the plunger 12. Particularly, with reference to Figure 3, the ratchet teeth 20 are preferably saw-tooth shaped having a ramped surface 28 and a shoulder stop 30. As shown in Figure 3, the ramped surfaces 28 of the ratchet teeth 20 on both the first and second surfaces 24, 26 are oriented to face in the same general direction. The shoulder stops 30 extend transversely from the first and second faces 24, 26, preferably at a substantially perpendicular orientation.

The plunger 12 may also have one or more rails 27 extending from the first face 24 and/or the second face 26. The rails 27 may be formed to slide through one or more corresponding shape-mating slots formed in the medical injector 10. The rails 27 may provide stability during use, particularly during translation of the plunger 12.

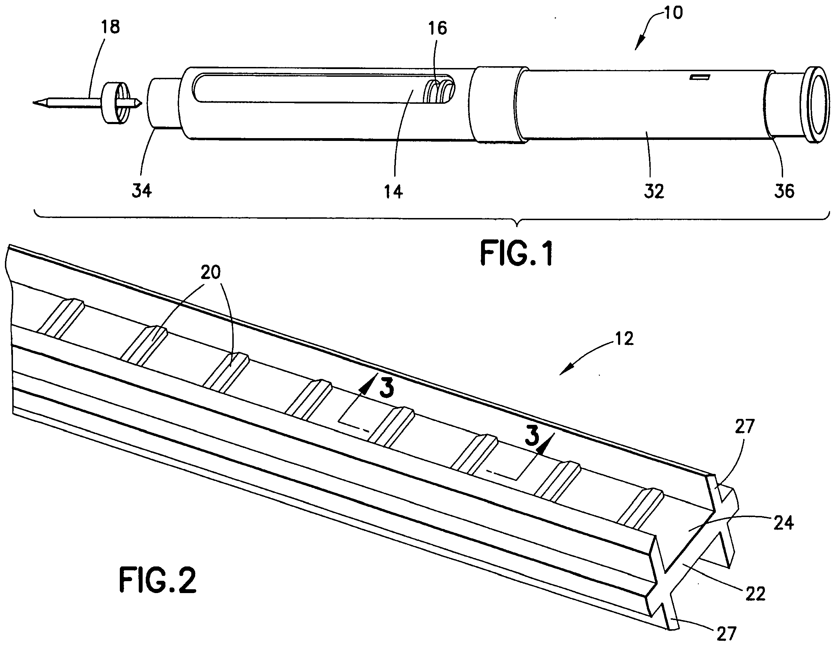

With reference to Figures 4-6, the plunger 12 is disposed in a body 32 of the medical injector 10. The body 32 includes a distal end 34, located to be directed toward a patient during an injection, and a proximal end 36, located to be away from a patient during an injection (Figure 1). During use, the medical injector 10 is configured to permit the plunger 12 to move unidirectionally therein in a distal direction toward the distal end 34, but not in a proximal direction toward the proximal end 36. To facilitate such unidirectional movement, at least one indexer 38 is provided formed to engage the plunger 12. The indexer 38 is configured to allow the plunger 12 to displace distally toward the distal end 34 of the body 32 but not proximally toward the proximal end 36 of the body 32.

The indexer 38 includes a deflectable pawl 40 which, as shown schematically in Figure 3, includes a ramped engagement surface 42 and an outward facing stop surface 44. The indexer 38 is outwardly deflectable to permit the engagement surface 42 to ascend the ramped surface 28 of an individual of the ratchet teeth 20 with the plunger 12 moving distally relative thereto. With sufficient distal movement, the indexer 38 bypasses the ratchet teeth 12, and under inherent resilience of the indexer 38, snaps inwardly such that the stop surface 44 is aligned with the shoulder stop 30. Preferably, the stop surface 44 is formed to be generally parallel to the shoulder stop 30. With rearward (proximal) movement of the plunger 12, the shoulder stop 30 and the stop surface 44 interferingly engage thus preventing proximal movement of the plunger 12. In a preferred embodiment, a pair of the indexers 38 are provided so as to act against the ratchet teeth 20 located on both the first and second faces 24, 26, as shown in Figures 4-6. It is further preferred that a pair of the indexers 38 be provided which are axially aligned thus providing a pinching effect to the plunger 12. This pinching effect may provide a stable holding force for the plunger 12.

The indexer 38 may be formed to be deflectable through inherent resilience, such as through material selection (e.g., being formed of a thermoplastic). In addition, or alternatively, the indexer 38 may include a cantilevered arm 46 which permits deflection of the associated pawl 40. The indexer 38 is formed to have a natural, unbiased state as shown in Figures 4-6, where the indexer 38 is positioned to act against the shoulder stop 30 of the ratchet teeth 20. The cantilevered arm 46 is formed with sufficient internal memory to provide the unbiased state for the indexer 38.

The medical injector 10 also includes an actuator 48 having an engagement portion 50 formed to engage one or more of the ratchet teeth 20. The engagement portion 50 preferably includes an engagement pawl 52 having a ramped engagement surface 54 and an outward facing stop surface 56 configured like the pawl 40 described above. Preferably, two of the engagement portions 50 are provided located to engage the ratchet teeth 20 located on the first and second faces 24, 26.

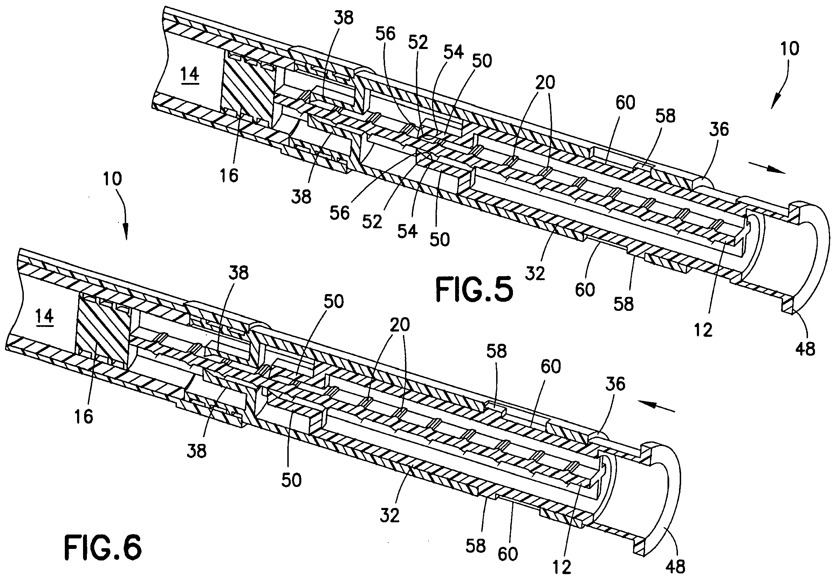

With reference to Figure 4, the plunger 12 is positioned to engage one of the stoppers 16. To cause actuation of the medical injector 10, as shown in Figure 5, the actuator 48 is moved to a ready state, with the engagement portion 50 moving proximally. The indexer 38 prevents proximal movement of the plunger 12, thus allowing the actuator 48 to move proximally relative to the plunger 12. With the plunger 12 being held stationary, and the engagement portion 50 moving proximally relative to the plunger 12, the engagement portion 50 bypasses one or more of the ratchet teeth 20. The actuator 48 is displaced sufficiently to achieve a ready state.

For actuation of the medical injector 10, the actuator 48 is displaced from the ready state with distal movement of the engagement portion 50. The engagement portion 50 engages one or more of the ratchet teeth 20, particularly with interfering engagement between the shoulder stop 30 and the stop surface 56. In particular, the engagement portion 50 engages the next distal ratchet tooth 20. Distal movement of the engagement portion 50 causes the plunger 12 to move distally therewith. Distal movement of the plunger 12, in turn, causes distal advancement of the stopper 16 in causing an injection to be administered. The ratchet teeth 20 are able to bypass the indexer 38 in the distal direction.

The size of a dose to be administered by the medical injector 10 is a function of the spacing between the ratchet teeth 20 and/or the amount of proximal displacement of the engagement portion 50 relative to the ratchet teeth 20 with the actuator 48 moving to a ready state. To create a fixed dose, one or more keys 58 may be defined on the medical injector 10 and/or the actuator 48 which are formed to nest within and slide along corresponding channels 60 formed in the medical injector 10 and/or the actuator 48. As shown in Figures 4-6, it is preferred that the keys 58 be formed on the actuator 48 and the channels 60 be formed in the medical injector 10. With reference to Figure 4, the keys 58 are at the distal end of the channel 60, prior to use. With proximal displacement of the actuator 48, the keys 58 are proximally advanced in the channels 60 to a proximal-most position corresponding to the ready state of the actuator 48. The length of the travel of the keys 58 in the channels 60 restricts the range of movement of the actuator 48 in defining the size of the dose administrable by the medical injector 10. As shown in Figures 5 and 6, the keys 58 are advanced distally with the actuator 48 during use to a distal-most position.

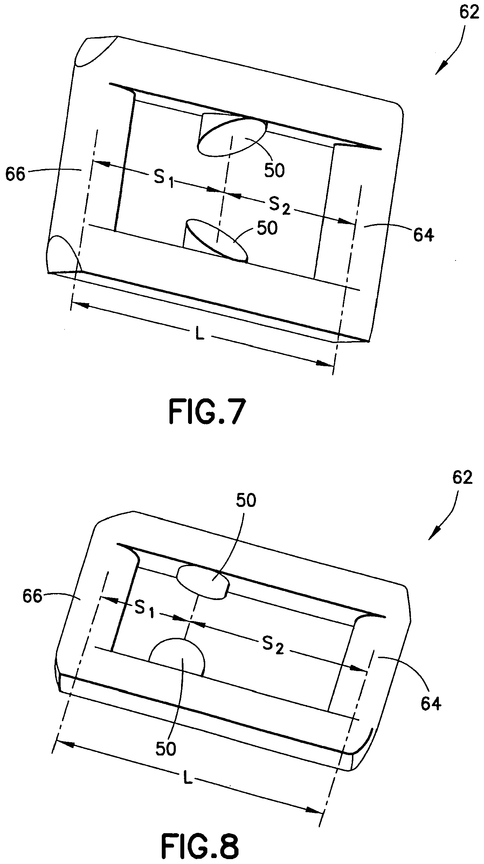

The actuator 48 shown in Figures 4-6 is a linear slide actuator which applies force directly to the plunger 12. As will be appreciated by those skilled in the art, the actuator 48 may be of various configurations. With reference to Figures 7-8, the actuator 48 may include a rocker 62 pivotally mounted thereto. The rocker 62 is frame-shaped, having a first end 64 for pivotal mounting to the actuator 48 and a second opposing end 66 for pivotally mounting to the medical injector 10. The engagement portion 50 is located between the first and second ends 64, 66. As shown in Figures 7 and 8, two of the engagement portions 50 may be provided to coact with the ratchet teeth 20 being located on the first and second faces 24, 26.

It is noted that the spacing between the first and second ends 64, 66 affects force transmission from the actuator 48 to the plunger 12 particularly in the generation of torque. The spacing L between the first and second ends 64, 66, as well as the spacing S1, S2 of the engagement portion 50 from the first and second ends 64, 66, affects how torque is generated and transmitted to the plunger 12.

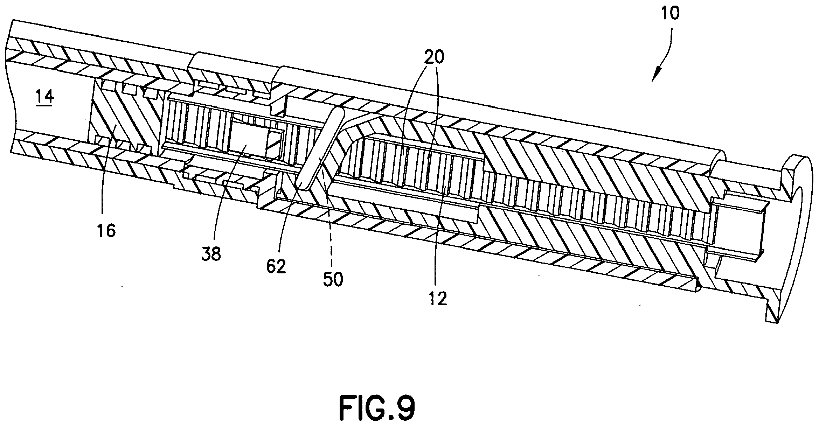

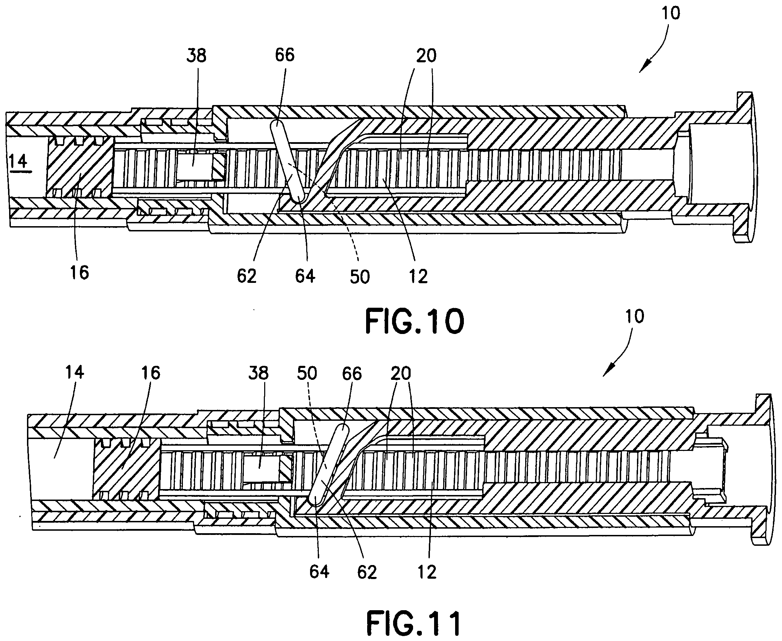

With reference to Figure 9, the actuator 48 is shown in an initial pre-use state with the rocker 62 being inclined distally. With reference to Figure 10, the actuator 48 has been advanced to the ready state with the rocker 62 having been drawn proximally with rotation about the second end 66 so as to be inclined in a proximal direction. During this movement, the engagement portion 50 bypasses one or more of the ratchet teeth 20 in the same manner as described above. As shown in Figure 11, the actuator 48 is displaced from the ready state to cause actuation of the medical injector 10. With displacement of the actuator 48 from the ready state, the rocker 62 is caused to advance distally about the second end 66 with the engagement portion 50 causing the plunger 12 to also advance distally. Dose size may be restricted both by the key 58/channel 60 arrangement described above and/or by the range of motion of the rocker 62.

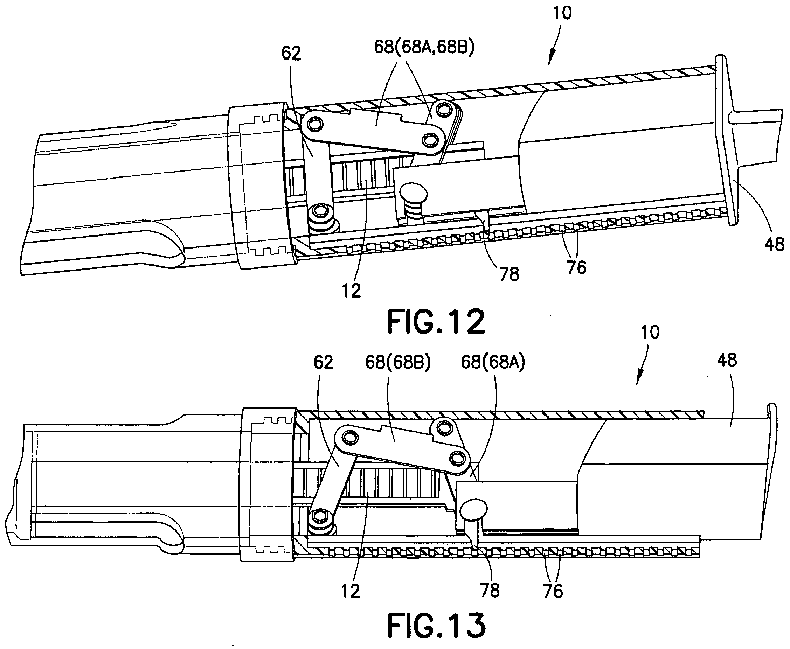

As will be appreciated by those skilled in the art, the rocker 62 may be directly coupled to the actuator 48, as shown in Figures 9-11. As will be appreciated by those skilled in the art, a multi-link arrangement may be used to couple the rocker 62 to the actuator 48. With reference to Figures 12 and 13, one or more links 68 may be connected between the rocker 62 and the actuator 48 to provide force for displacement thereof. Any arrangement of the links 68 may be utilized which transmits force from the actuator 48 to the rocker 62. As shown in Figures 12 and 13, two of the links 68 (68A, 68B) are utilized with the link 68A being pivotally connected to the actuator 48 and pivotally connected to the link 68B, and with the link 68B being pivotally connected to the link 68A and pivotally connected to the rocker 62. The links 68A, 68B collectively transmit force to the rocker 62.

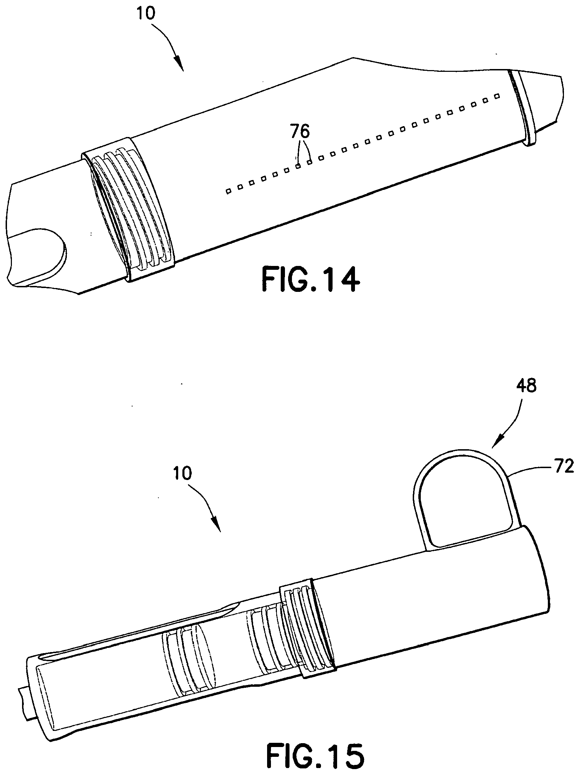

With reference to Figures 15-17, the actuator 48 may be arranged to be non-linearly displaced. As shown in Figures 15-17, the actuator 48 may be formed to pivot about a fulcrum 70. With the actuator 48 pivoting outwardly from the medical injector 10 about the fulcrum 70, the engagement portion 50 is caused to be displaced proximally. Conversely, inward pivoting of the actuator 48 about the fulcrum 70 causes distal displacement of the engagement portion 50. The engagement portion 50 coacts with the plunger 12 in the same manner as described above.

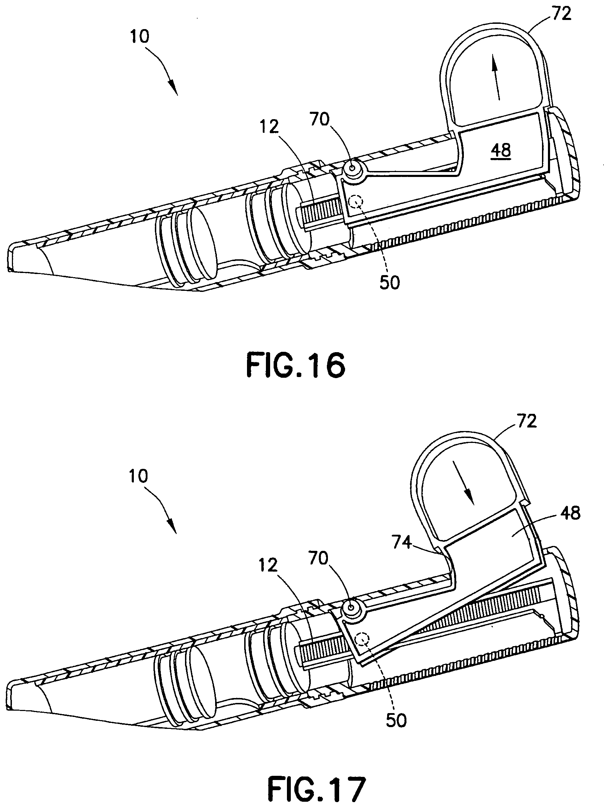

With reference to Figures 15 and 16, the medical injector 10 is shown in a pre-use state. To facilitate handling of the actuator 48, a grip ring or pad 72 may be provided which extends radially outwardly from the medical injector 10 to facilitate displacement of the actuator 48. To prepare for use, as shown by the arrow in Figure 16, the actuator 48 is pivoted to a ready state as shown in Figure 17. To cause actuation of the medical injector 10, the actuator 48 is pivoted inwardly from the ready state, as shown by the arrow in Figure 17.

The size of the dose may be fixed with the actuator 48 being pivotable by limiting the range of rotation of the actuator 48. A portion 74 of the medical injector 10 may be configured to limit the range of rotation of the actuator 48, particularly outward rotation, such limited range corresponding to the ready state.

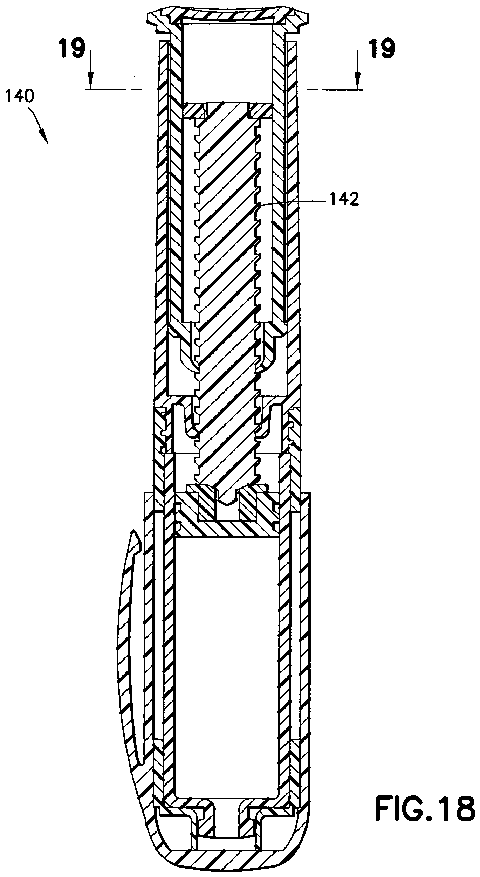

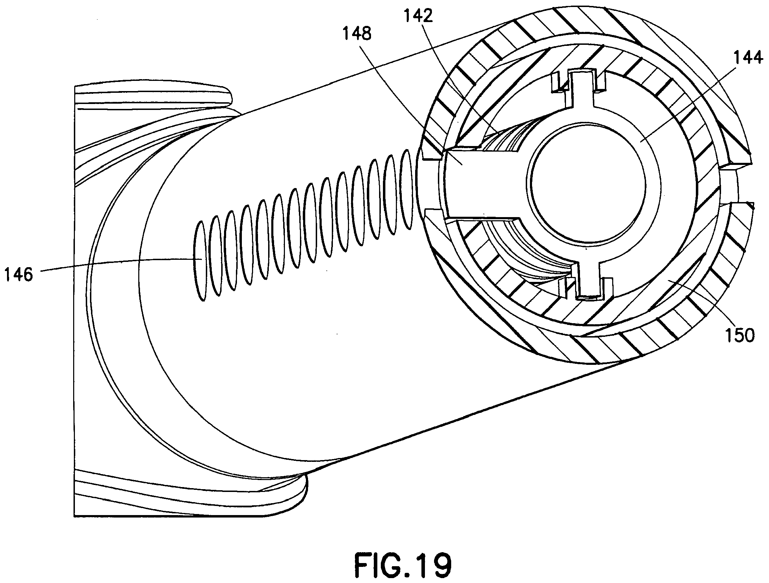

In the above-described embodiments, the plunger 12 is substantially flat. In contrast, in the following embodiments, the plungers are substantially cylindrical. For example, in the medical injector 140 shown in figures 18 and 19, the plunger 142 is substantially cylindrical and the ratchet teeth extend circumferentially around the plunger 142. In such an embodiment, the orientation of the plunger is not critical, thereby easing assembly of the device. In addition, as shown in figures 18 and 19, a pointer or indicator flag 144 connected to the plunger 142 is visible to a user through indicating holes 146 to indicate the dosage. The pointer 144 engages an axial slot 148 in the actuator 150 to provide an alignment feature.

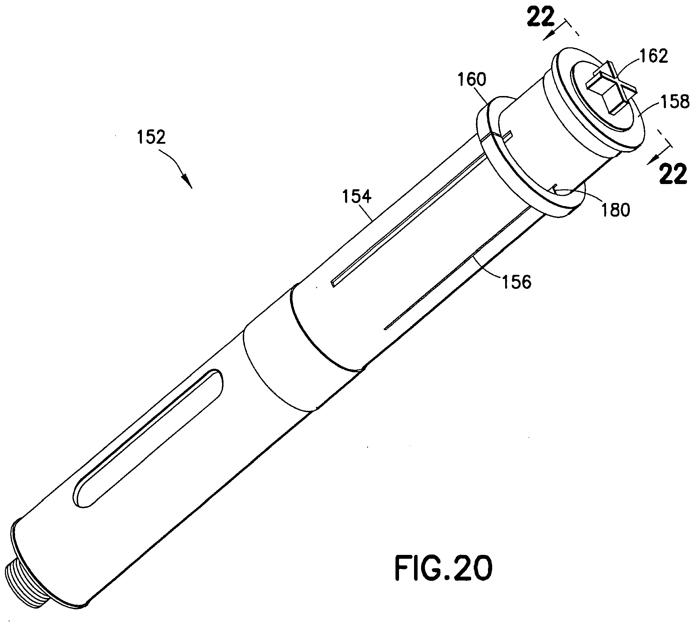

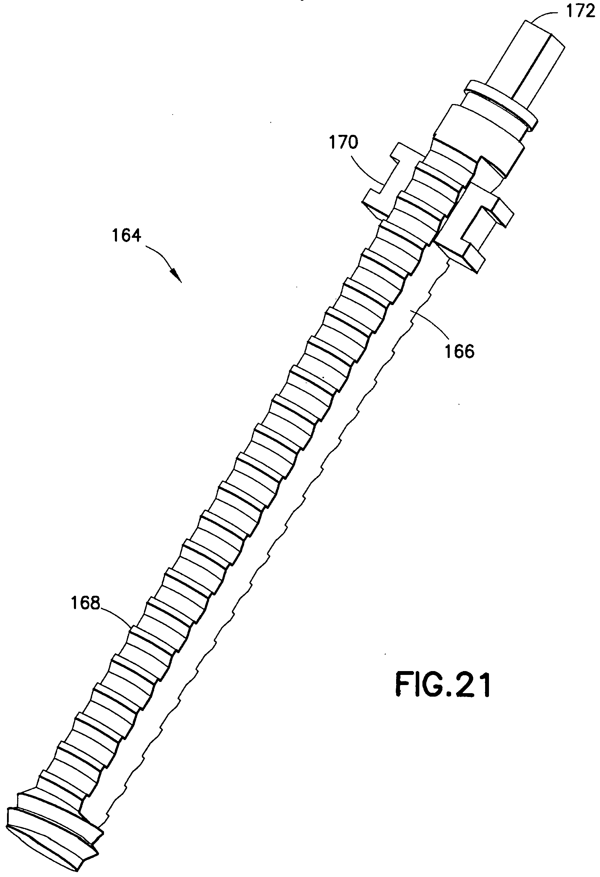

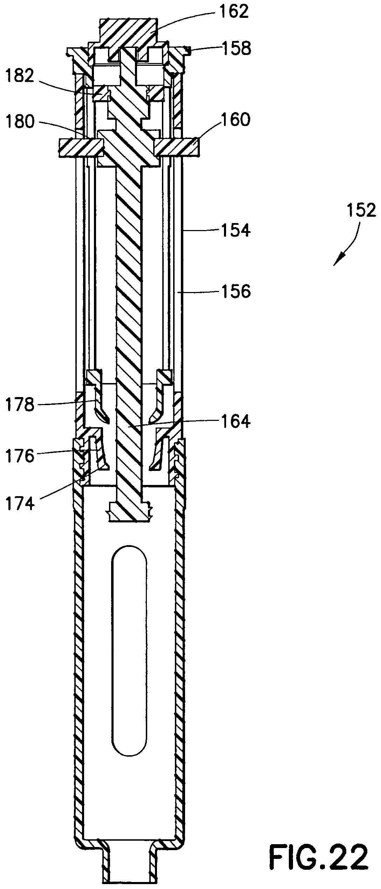

Figure 20 illustrates a medical injector 152 according to another embodiment of the present invention, figure 21 is a perspective view of a plunger usable with the medical injector 152, and figure 22 is a cross-sectional view of the medical injector 152. As shown in figures 20 and 22, the medical injector 152 includes a body 154 having axial slots 156 therein, an actuator 158, a plunger slider or ring slider 160 with arms extending through the slots 156, and a mode selector 162. According to one embodiment, the ratchet teeth on the plunger are circumferentially discontinuous. Put another way, at least one portion of the plunger is axially free of ratchet teeth. Figure 21 illustrates another embodiment of a cylindrical plunger 164 in which the plunger 164 is frusto-cylindrical. In other words, the plunger 164 has a pair of flat sides 166. As with the plunger 12, the plunger 164 also has a plurality of spaced-apart ratchet teeth 168 disposed along the length thereof. The plunger 164 also has a pair of ring slider connectors 170 disposed on the flat sides 166 and a mode selector connector 172 disposed at a proximal end of the plunger 164. The illustrated mode selector connector 172 is substantially square. One skilled in the art will appreciate that other shapes may be used without departing from the scope of the present invention. For example, the mode selector connector 172 may be triangular, rectangular, pentagonal, or hexagonal, or may have more sides.

The mode selector 162 has a recess corresponding to the mode selector connector 172 to engage the mode selector connector 172. While in the illustrated embodiment, the male protrusion (mode selector connector 172) is disposed at the proximal end of the plunger 166 and the corresponding female recess is disposed on the distal end of the mode selector 162, one skilled in the art will appreciate that the male protrusion may be disposed on the distal end of the mode selector 162 and the corresponding female recess may be disposed on the proximal and of the plunger 166 without departing from the scope of the present invention. The mode selector 162 is rotatably disposed with respect to the actuator 158, and thereby, as described in greater detail below, provides an interface for a user to rotate the plunger 166.

Similar to the embodiments described previously, in one orientation, the ratchet teeth 168 of the plunger 166 engage the pawls 174 of the indexer 176 and the engagement pawls 178 of the actuator 158. But because of the design of the plunger 164 (including the flat sides 166), the plunger 164 is rotatable with respect to the body 154. Accordingly, using the mode selector 162, a user can rotate the plunger 164 to an orientation in which the ratchet teeth 168 are disengaged from the pawls 174 and the engagement pawls 178, and the flat sides 166 are aligned with the pawls 174 and the engagement pawls 178, as shown in figure 22. Such an alignment permits selective, free distal and proximal displacement of the plunger 164. This feature is useful, for example, for reconstituting a lyophilized medicament, which will be discussed in greater detail below.

According to one embodiment, the ring slider connectors 170 disposed on the flat sides 166 of the plunger 164 selectively engage the arms 180 of the ring slider 160 that extend inwardly through the slots 156 of the body 154. For example, when a user rotates the mode selector 162 to disengage the ratchet teeth 168 from the pawls 174 and the engagement pawls 178, the ring slider connectors 170 rotate into engagement with the arms 180 of the ring slider 160. The user then can use the ring slider 162 to control displacement of the plunger 164.

As shown in figure 22, medical injector 152 also includes a pointer 182 for indicating the dosage, similar to the pointer 144. According to another embodiment, in which the axial slots 156 are circumferentially enlarged to accommodate rotation of the ring slider 160, the plunger 164 is fixedly connected to the ring slider 160, which serves as a dosage indicator.

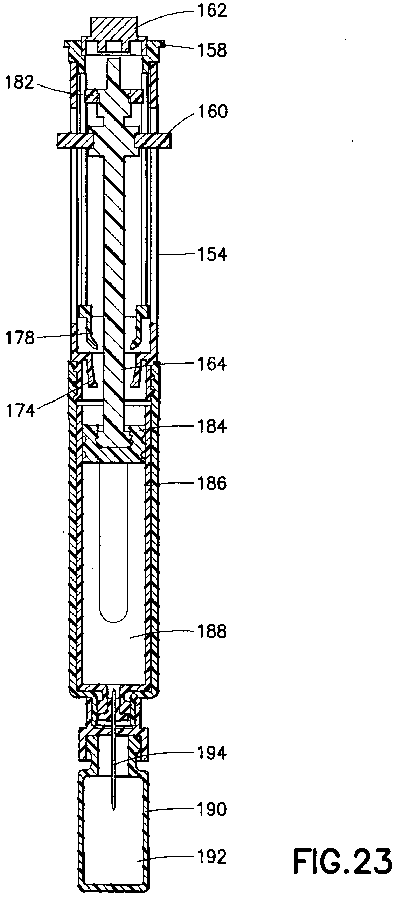

Reconstitution of a lyophilized medicament will now be described with reference to figure 23. The stopper 184 is disposed on the distal end of the plunger 164 inside a cartridge 186 containing a diluent 188 therein. The medicament container 190 with a lyophilized medicament 192 disposed therein is connected to a distal end of the cartridge 186 with a double-ended needle 194 communicating between interior of the medicament container 190 and the cartridge 186. After the user rotates the mode selector 162 to disengage the ratchet teeth 168 from the pawls 174 and the engagement pawls 178, the user distally slides the ring slider 160, thereby distally displacing the plunger 164 and the stopper 184, and expelling the diluent 188 into the medicament container 190. Subsequent to the reconstitution of the lyophilized medicament 192, the user proximally slides the ring slider 160, thereby proximally displacing the plunger 164 and the stopper 184, and drawing the reconstituted medicament into the cartridge 186. Thereafter, the user disconnects the medicament container 190 and rotates the mode selector 162 to reengage the ratchet teeth 168 with the pawls 174 and the engagement pawls 178. At this point, the reconstituted medicament can be injected as described above.

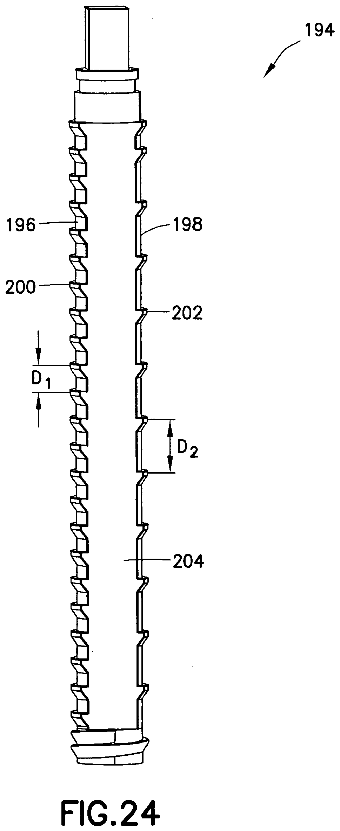

In the medical injector employing a ratchet plunger similar to those described above, discrete dosage settings are determined by the pitch of (or spacing between) the ratchets. Figure 24 is a perspective view of the plunger 194 in accordance with another embodiment of the present invention in which a user may select one of a plurality of predetermined ratchet spacings. As shown in figure 24, the plunger 194 has two sides with and 196 and 198 with respective pluralities of spaced-apart ratchet teeth 200 and 202 disposed along the length thereof. The distance between ratchet teeth 200 is shown as D₁ and the distance between ratchet teeth 202 is shown as D₂, which is greater than D₁. The plunger 194 is rotatable with respect to the body 154 and the actuator 158 so that a user can select a desired spacing between ratchet teeth to engage the pawls 174 and the engagement pawls 178. According to one embodiment, the mode selector 162 and the actuator 158 have demarcations corresponding to the various spacings of the ratchet teeth.

According to one embodiment, the plunger 194 also has a flat side 204 so that the user can rotate the plunger 194 to an orientation in which the ratchet teeth 168 are disengaged from the pawls 174 and the engagement pawls 178, and the flat side 204 is aligned with the pawls 174 and the engagement pawls 178. Thus, the plunger 194 can also be employed to reconstitute a lyophilized medicament.

In addition, although the plunger 194 is illustrated as having two sides with respective pluralities of spaced-apart ratchet teeth, one skilled in the art will appreciate that the plunger 194 may have 3, 4, 5, 6, 7, 8, 9, 10, or more sides with respective pluralities of differently-spaced-apart ratchet teeth to provide a greater selection for a user without departing from the scope of the present invention. Additionally, although the pawls 174 and the engagement pawls 178 are illustrated as being opposing pairs, a single pawl 174 and a single engagement pawl 178 may be employed to accommodate a greater number of sides with differently spaced ratchet teeth on the plunger 194 without departing from the scope of the present invention.

Although only a few embodiments of the present invention have been shown and described, the present invention is not limited to the described embodiments. Instead, it will be appreciated by those skilled in the art that changes may be made to these embodiments without departing from the scope of the present invention, which is defined by the appended claims.

## Claims

1. A medical injector (152), comprising:
a body (154) having a distal end and a proximal end;
a displaceable plunger (164, 194) disposed in an interior of the body, the plunger having a plurality of spaced-apart ratchet teeth (168, 200, 202) disposed along the length thereof;
at least one indexer (176) having a pawl (174) formed to engage the ratchet teeth of the plunger, wherein the indexer is configured to allow the plunger to displace distally toward the distal end of the body but not proximally toward the proximal end of the body; and
an actuator (158) having an engagement portion (178) formed to engage one or more of the ratchet teeth, wherein the actuator is displaceable to a ready state, the engagement portion being displaced proximally relative to the plunger upon the actuator being displaced to the ready state, the indexer preventing proximal movement of the plunger thereby allowing the engagement portion to bypass one or more of the ratchet teeth upon the actuator being displaced to the ready state, and wherein the actuator is displaceable from the ready state to cause actuation of the medical injector, the displacement from the ready state causing distal displacement of the engagement portion with the engagement portion engaging one or more of the ratchet teeth and causing distal displacement of the plunger;
**characterized in that**:
the medical injector further comprises a plunger slider (160) disposed on an exterior of the body to be operable by a user;
wherein the plunger is slidably displaceable relative to the body, and rotatable relative to the body to an orientation in which the ratchet teeth are engaged or disengaged from the indexer and the actuator; and
wherein the body comprises axial slots (156) and the plunger slider has arms (180) extending through the axial slots to engage the plunger, the plunger slider arms being slidable along the axial slots.

2. The medical injector according to claim 1, wherein the plunger comprises at least one flat side (166, 204) alignable with the indexer and the actuator, to disengage the ratchet teeth from the indexer and the actuator.

3. The medical injector according to claim1, wherein the plunger is rotatable relative to the plunger slider to selectively engage and disengage the plunger slider.

4. The medical injector according to claim 1, further comprising a mode selector (162) rotatable relative to the actuator and the body and being connected to the plunger to control rotation of the plunger relative to the body.

5. The medical injector according to claim 4, wherein the mode selector comprises one of a protrusion and a recess having a shape corresponding to the protrusion, and the plunger comprises the remaining one of the protrusion and the recess having a shape corresponding to the protrusion.

6. A method of reconstituting a lyophilized medicament using a medical injector (152)of claim 1, the method comprising:
connecting a medicament container containing the lyophilized medicament to an end of the body so that a double ended needle communicates between the medicament container and a diluent containing cartridge disposed within the body;
**characterized in that** the method further comprises:
rotating the plunger to disengage ratchet teeth (168, 200, 202) on the plunger from pawls (174) of the body to engage an arm of a plunger slider (160) with a slider connector;
displacing the plunger to eject diluent into the medicament container by distally sliding the plunger slider;
displacing the plunger to draw reconstituted medicament into the cartridge by proximally sliding the plunger slider; and
rotating the plunger to engage the ratchet teeth of the plunger with the body.

## Patentansprüche

1. Medizinischer Injektor (152), mit:
einem Körper (154) mit einem distalen Ende und einem proximalen Ende;
einem verschiebbaren Kolben (164,194), der in einem Innenraum des Körpers angeordnet ist, wobei der Kolben mehrere voneinander beabstandete Ratschenzähne (168,200,202) aufweist, die entlang seiner Länge angeordnet sind;
mindestens einem Schrittgetriebe (176) mit einer Sperrklinke (174), die zum Zusammengriff mit den Ratschenzähnen des Kolbens ausgebildet ist, wobei das Schrittgetriebe derart konfiguriert ist, dass es eine Verschiebung des Kolbens distal zu dem distalen Ende des Körpers hin, jedoch nicht proximal zu dem proximalen Ende des Körpers hin zulässt; und
einem Aktuator (158) mit einem Zusammengriffsabschnitt (178), der zum Zusammengreifen mit einem oder mehreren der Ratschenzähne ausgebildet ist, wobei der Aktuator in einen Bereitschaftszustand verschiebbar ist, wobei der Zusammengriffsabschnitt relativ zu dem Kolben proximal versetzt ist, wenn der Aktuator in den Bereitschaftszustand worden verschoben ist, wobei das Schrittgetriebe eine proximale Bewegung des Kolbens verhindert, wodurch dem Zusammengriffsabschnitt ermöglicht wird, sich an einem oder mehreren der Ratschenzähne vorbei zu bewegen, wenn der Aktuator in den Bereitschaftszustand verschoben worden ist, und wobei der Aktuator aus dem Bereitschaftszustand heraus verschiebbar ist, um eine Betätigung des medizinischen Injektors zu bewirken, wobei die Verschiebung aus dem Bereitschaftszustand heraus eine distale Verschiebung des Zusammengriffsabschnitts bewirkt, wenn der Zusammengriffsabschnitt mit einem oder mehreren der Ratschenzähne zusammengreift und eine distale Verschiebung des Kolbens bewirkt;
**dadurch gekennzeichnet, dass**:
der medizinische Injektor ferner ein Kolben-Gleitbetätigungsteil (160) aufweist, das an einem Außenbereich des Körpers derart angeordnet ist, dass es von einem Benutzer betätigbar ist;
wobei der Kolben relativ zu dem Körper gleitend verschiebbar ist und relativ zu dem Körper in eine Orientierung drehbar ist, in der die Ratschenzähne mit dem Schrittgetriebe und dem Aktuator zusammengreifen oder aus diesen ausgerückt sind; und
wobei der Körper axiale Schlitze (156) aufweist und das Kolben-Gleitbetätigungsteil Arme (180) aufweist, die sich durch die axialen Schlitze erstrecken, um mit dem Kolben zusammenzugreifen, wobei die Arme des Kolben-Gleitbetätigungsteils entlang der axialen Schlitze gleitbar sind.

2. Medizinischer Injektor nach Anspruch 1, bei dem der Kolben mindestens eine flache Seite (166,204) aufweist, die mit dem Schrittgetriebe und dem Aktuator ausrichtbar ist, um die Ratschenzähne aus dem Schrittgetriebe und dem Aktuator ausrücken zu lassen.

3. Medizinischer Injektor nach Anspruch 1, bei dem der Kolben relativ zu dem Gleitbetätigungsteil drehbar ist, um selektiv mit dem Gleitbetätigungsteil zusammenzugreifen und aus diesem auszurücken.

4. Medizinischer Injektor nach Anspruch 1, ferner mit einem Modus-Selektor (162), der relativ zu dem Aktuator und dem Körper drehbar ist und mit dem Kolben verbunden ist, um die Drehung des Kolbens relativ zu dem Körper zu steuern.

5. Medizinischer Injektor nach Anspruch 4, bei dem der Modus-Selektor einen bzw. eine aus einem Vorsprung oder einer Ausnehmung mit einer dem Vorsprung entsprechenden Form aufweist, und der Kolben den bzw. die andere aus dem Vorsprung oder der Ausnehmung mit einer dem Vorsprung entsprechenden Form aufweist.

6. Verfahren zum Rekonstituieren eines lyophilisierten Medikaments mittels eines medizinischen Injektors (152) nach Anspruch 1, wobei das Verfahren umfasst:
Verbinden eines Medikamentenbehälters, der das lyophilisierte Medikament enthält, mit einem Ende des Körpers, so dass eine doppelendige Nadel eine Verbindung zwischen dem Medikamentenbehälter und einer in dem Körper angeordneten Cartridge bildet, die ein Verdünnungsmittel enthält;
**dadurch gekennzeichnet, dass** das Verfahren ferner umfasst:
Drehen des Kolbens derart, dass an dem Kolben angeordnete Ratschenzähne (168,200,202) aus Sperrzähnen (174) des Körpers ausrücken, um einen Arm eines Kolben-Gleitbetätigungsteils (160) mit einem Gleitbetätigungsteil-Konnektor in Zusammengriff zu bringen;
Verschieben des Kolbens zum Ausstoßen von Verdünnungsmittel in den Medikamentenbehälter durch distales gleitendes Bewegen des Kolben-Gleitbetätigungsteils;
Verschieben des Kolbens zum Einsaugen rekonstituierten Medikaments in die Cartridge durch proximales gleitendes Bewegen des Kolben-Gleitbetätigungsteils; und
Drehen des Kolbens, um die Ratschenzähne des Kolbens in Zusammengriff mit dem Körper zu bringen.

## Revendications

1. Injecteur médical (152), comprenant :
un corps (154) ayant une extrémité distale et une extrémité proximale ;
un piston mobile (164, 194) disposé à l'intérieur du corps, le piston ayant une pluralité de dents triangulaires espacées (168, 200, 202) disposées sur sa longueur ;
au moins un repère (176) ayant un cliquet (174) formé pour s'engager avec les dents triangulaires du piston, où le repère est configuré pour permettre au piston de se déplacer de manière distale vers l'extrémité distale du corps mais de ne pas se déplacer de manière proximale vers l'extrémité proximale du corps ; et
un actionneur (158) ayant une partie d'engagement (178) formée pour s'engager avec une ou plusieurs des dents triangulaires, où l'actionneur peut se déplacer vers un état prêt, la partie d'engagement se déplaçant de manière proximale par rapport au piston lors du déplacement de l'actionneur vers l'état prêt, le repère empêchant un mouvement proximal du piston permettant ainsi à la partie d'engagement de contourner une ou plusieurs des dents triangulaires lors du déplacement de l'actionneur vers l'état prêt, et où l'actionneur peut se déplacer de l'état prêt pour entraîner l'actionnement de l'injecteur médical, le déplacement de l'état prêt entraînant un déplacement distal de la partie d'engagement avec la partie d'engagement s'engageant avec une ou plusieurs des dents triangulaires et entraînant un déplacement distal du piston ;
**caractérisé en ce que** :
l'injecteur médical comprend en outre un coulisseau de piston (160) disposé à l'extérieur du corps pour pouvoir être actionné par un utilisateur ;
dans lequel le piston peut se déplacer en coulissement par rapport au corps, et peut tourner par rapport au corps à une orientation où les dents triangulaires sont engagées avec ou désengagées du repère et de l'actionneur ; et
dans lequel le corps comprend des fentes axiales (156) et le coulisseau de piston a des bras (180) s'étendant à travers les fentes axiales pour s'engager avec le piston, les bras de coulisseau de piston pouvant coulisser le long des fentes axiales.

2. Injecteur médical selon la revendication 1, dans lequel le piston comprend au moins un côté plat (166, 204) pouvant être aligné avec le repère et l'actionneur, pour désengager les dents triangulaires du repère et de l'actionneur.

3. Injecteur médical selon la revendication 1, dans lequel le piston peut tourner par rapport au coulisseau de piston pour engager et désengager sélectivement le coulisseau de piston.

4. Injecteur médical selon la revendication 1, comprenant en outre un sélecteur de mode (162) pouvant tourner par rapport à l'actionneur et au corps et étant relié au piston pour commander la rotation du piston par rapport au corps.

5. Injecteur médical selon la revendication 4, dans lequel le sélecteur de mode comprend l'un(e) d'une saillie et d'un évidement présentant une forme correspondant à la saillie, et le piston comprend l'autre de la saillie et de l'évidement présentant une forme correspondant à la saillie.

6. Procédé de reconstitution d'un médicament lyophilisé en utilisant un injecteur médical (152) de la revendication 1, le procédé comprenant le fait :
de relier un récipient de médicament contenant le médicament lyophilisé à une extrémité du corps de sorte qu'une aiguille à double extrémité communique entre le récipient de médicament et une cartouche contenant un diluant disposée dans le corps ;
**caractérisé en ce que** le procédé comprend en outre le fait :
de faire tourner le piston pour désengager les dents triangulaires (168, 200, 202) sur le piston des cliquets (174) du corps pour engager un bras d'un coulisseau de piston (160) avec un raccord de coulisseau ;
de déplacer le piston pour éjecter un diluant dans le récipient de médicament par coulissement distal du coulisseau de piston ;
de déplacer le piston pour aspirer le médicament reconstitué dans la cartouche par coulissement proximal du coulisseau de piston ; et
de faire tourner le piston pour engager les dents triangulaires du piston avec le corps.
